# EUROPEAN PATENT SPECIFICATION

(11) **EP 3 525 850 B1**
(45) Date of publication and mention of the grant of the patent: **30.12.2020**
(21) Application number: 17772069.5
(22) Date of filing: 25.09.2017
(51) Int. Cl.: A61M 5/32

(54) **SUB-ASSEMBLY FOR A MEDICAMENT DELIVERY DEVICE**
KAPPENANORDNUNG FÜR EINE MEDIKAMENTENFREISETZUNGSVORRICHTUNG
ENSEMBLE FORMANT CAPUCHON POUR DISPOSITIF D'ADMINISTRATION DE MÉDICAMENTS

(30) Priority: 13.10.2016 EP 16193817
(43) Date of publication of application: 21.08.2019
(73) Proprietor: SHL Medical AG, 6302 Zug (CH)
(72) Inventor: WANG, Hsuan, Taoyuan City 338 (TW); CHOU, Jung-Chien, Changhua 520 (TW); LEE, Wen-Yen, Taoyuan City 334 (TW)
(86) International application number: PCT/EP2017/074258
(87) International publication number: WO 2018/069032

(56) References cited:
- EP-A1- 2 923 716
- WO-A1-2014/154498
- WO-A1-2015/110532
- WO-A1-2016/055334

## Description

### TECHNICAL FIELD

The present disclosure generally relates to medical devices. In particular, it relates to a sub-assembly for a medicament delivery device, to a medicament delivery device comprising the sub-assembly, and to a method of assembling a medicament delivery device.

### BACKGROUND

Medicament delivery devices, such as injectors and inhalers, typically comprise a housing in which a medicament container containing a medicament is to be arranged. Upon activation of the medicament delivery device, the medicament is expelled through a medicament delivery member, for example a needle or a nozzle.

In order to protect and to keep the medicament delivery member sterile, the medicament delivery member may be provided with a delivery member shield, or sheath, such as a Flexible Needle Shield (FNS) or a Rigid Needle Shield (RNS). The delivery member shield may thus be attached to the medicament container to cover the delivery member.

Moreover, the medicament delivery device may comprise a removable cap which is mounted to the proximal end of the housing, i.e. that end which is placed towards the injection site during medicament delivery, of the medicament delivery device, or to the proximal end of the medicament container. The removable cap has the function of providing mechanical protection of the medicament delivery member while attached to the housing or medicament container, and to remove the delivery member shield when the cap is removed from the housing.

An example of a device for removing a needle shield from an auto-injector is disclosed in WO2015110532 A1. WO2015110532 A1 discloses an auto-injector having a connector for connecting a needle cover to a removable cap. The connector has a plurality of legs spaced symmetrically away from one another about a central hub. The legs have an elastic nature and aid in securing the needle cover and/or rigid needle shield to a cap insert and hence to the removable cap. The needle cover and/or needle shield are secured together through upper, internally facing barbs protruding from the first legs. The upper, internally facing barbs include tips that point toward the forward end of the connector. These barbs are shaped to engage the needle cover and/or rigid needle shield when the needle cover and/or rigid needle shield is fitted within the connector. The barb tips apply opposing force with respect to one another when they engage the needle cover and/or rigid needle shield when the needle cover and/or rigid needle shield is fitted within the connector.

WO 2016/055334 discloses a medicament delivery device for injection of a medicament. The medicament delivery device comprises a housing, a medicament delivery member cover, which medicament delivery member cover (7) has a cap-fitting portion. The delivery member cover is moveable relative to the housing from an extended position in which the cap-fitting portion extends from the housing, to a retracted position in which the delivery member cover is biased towards the extended position end. A cap is arranged to receive the cap-fitting portion. The delivery member cover and the cap comprise a tamper evidence mechanism, which is activated by removal of the cap from the cap-fitting portion.

WO 2014/154498 discloses a front cap for a medicament delivery device with a proximal end and a distal end. The front cap is configured to be releasably connected to the medicament delivery device and comprises a shield grabber support and a shield grabber for connecting the front cap to an outer surface of a needle shield of a syringe. The syringe is positioned within the medicament delivery device. The shield grabber is coupled to the shield grabber support such that said shield grabber is axially moveable relative to the shield grabber support.

EP 2 923 716 discloses a cap for a medicament container with a protective needle sheath removably disposed on a needle. The cap comprises a distal face; at least one compliant sheath removal beam extending in a proximal direction from the distal face and defining a space for receiving a protective needle sheath. The at least one compliant sheath removal beam includes at least one ledge adapted to engage the protective needle sheath. The at least one compliant sheath removal beam is disposed approximately perpendicular to the distal face in a first position for engaging the protective needle sheath and is disposed at a non-approximately perpendicular angle to the distal face in a second position for receiving the protective needle sheath.

### SUMMARY

According to the design disclosed in WO2015110532 A1, the legs of the connector are flexed towards each other as soon as the connector is placed in the cap insert. This bending of the legs renders it more difficult to insert the needle cover/rigid needle shield into the connector, thereby making assembly more difficult. Moreover, there is a risk that the needle sheath will make contact with the barbs during insertion. Such contact is undesirable because the forces thus exerted onto the needle shield may cause movement of the needle shield relative to the needle which it encloses, which could potentially cause coring, i.e. that the needle becomes filled with debris from the needle shield, and/or that the needle is bent.

In view of the above, a general object of the present disclosure is to provide a cap assembly for a medicament delivery device which solves or at least mitigates the problems of the prior art.

There is hence according to a first aspect of the present disclosure provided a cap assembly for a medicament delivery device, comprising: a cap having a tubular body provided with a distal opening, the cap being configured to be mounted to a proximal end of a medicament delivery device, a tubular remover configured to be received in the distal opening of the cap, the remover having flexible radially inwards extending grippers configured to engage with a delivery member shield, and a tubular remover insert configured to be received by the remover and configured to be fixedly attached to the cap to prevent axial displacement of the remover insert relative to the cap, wherein the remover is axially displaceable relative to the cap and relative to the remover insert, from a first position in which the grippers are pressed radially outwards by an outer surface of the remover insert, to a second position located distally relative to the first position, in which second position the grippers are arranged distally beyond the outer surface of the remover insert, enabling the grippers to flex radially inwards to allow engagement with a delivery member shield.

The remover insert is thus configured to flex the grippers radially outwards when the remover is in the first position. The remover is in the first position when the cap assembly is attached to a medicament delivery device. Thus, as long as the cap is mounted to a medicament delivery device, or to a body thereof, the grippers will be flexed radially outwards, allowing a delivery member shield to be inserted into the remover and the remover insert without physically contacting the grippers. Since there is generally no contact between the delivery member shield and the grippers at this stage, there is no risk of coring, and there is no risk that the delivery member becomes bent. When the cap assembly is being removed from a medicament delivery device, there will be a relative movement between the remover and the remover insert, causing the grippers to be released from their radially outwardly pressed position. The grippers will thereby be able to engage or grip a medicament delivery shield received by the tubular remover.

According to one embodiment the remover has an outer surface provided with a first radial protrusion.

The first radial protrusion is configured to engage with a delivery member cover of a medicament delivery device or sub-assembly thereof, when the cap assembly has been assembled with a medicament delivery device or a housing of a medicament delivery device. When the cap assembly is being removed from the medicament delivery device or housing, the remover will initially maintain its axial position relative to the delivery member cover due to the first radial protrusion, while the cap and the remover insert are moved in the proximal direction. The remover will thus move from the first position towards the second position, causing the grippers to flex radially inwards and engage with the delivery member shield.

According to one embodiment the first radial protrusion extends in the circumferential direction along the outer surface of the remover.

According to one embodiment the outer surface of the remover is provided with a second radial protrusion.

The second radial protrusion is configured to be arranged proximally relative to a proximal end of the delivery member cover of a medicament delivery device or a sub-assembly thereof, when the cap assembly has been assembled with a medicament delivery device or a housing of a medicament delivery device. The second radial protrusion is configured to engage with a proximal edge or rim of the delivery member cover in case the cap assembly e.g. by accident is subjected to a force that would push it further in the distal direction after it has been properly assembled with a medicament delivery device or a housing. Movement of the cap assembly is thus restricted in the distal direction.

According to one embodiment the second radial protrusion is located proximally relative to the first radial protrusion.

According to one embodiment the second radial protrusion extends in the circumferential direction along the outer surface of the remover.

According to one embodiment the remover has a proximal end portion provided with a flange.

According to one embodiment the cap has a base member defining a proximal end of the cap, and a radial surface arranged distally from the base member, which base member and radial surface delimit a remover movement space, wherein the flange of the remover is configured to be arranged in the remover movement space, thereby enabling axial movement of the flange between the base member in which the remover is in the first position and the radial surface in which the remover is in the second position.

According to one embodiment the base member is configured to be attached to the tubular body of the cap, and wherein the remover insert is configured to be fixedly attached to the base member.

There is according to a second aspect of the present disclosure provided sub-assembly for a medicament delivery device, comprising: a housing, a delivery member cover configured to be received by the housing and configured to move axially relative to the housing, and a cap assembly according to the first aspect.

According to one embodiment the cap assembly is configured to be mounted onto a proximal end of the housing, and wherein the remover is configured to be received by the delivery member cover.

According to one embodiment the first radial protrusion is configured to engage with a proximal end portion of the delivery member cover such that proximal movement of the cap causes distal movement of the remover relative to the cap, from the first position to the second position thereof.

According to one embodiment the second radial protrusion is configured to be arranged proximally from a proximal end of the delivery member cover when the cap assembly is mounted to the delivery member cover, to restrict distal movement of the remover relative to the delivery member cover.

There is according to a third aspect of the present disclosure provided a medicament delivery device comprising a sub-assembly according to the second aspect.

There is according to a third aspect provided a method of assembling a medicament delivery device, comprising: a) providing a sub-assembly comprising: a cap having a tubular body provided with a distal opening, a tubular remover received in the distal opening of the cap, the remover having flexible radially inwards extending grippers configured to engage with a delivery member shield, and a tubular remover insert received by the remover and fixedly attached to the cap to prevent axial displacement of the remover insert relative to the cap, wherein the remover is axially displaceable relative to the cap and relative to the remover insert, from a first position in which the grippers are pressed radially outwards by an outer surface of the remover insert, to a second position located distally relative to the first position, in which second position the grippers are arranged distally beyond the outer surface of the remover insert, enabling the grippers to flex radially inwards to allow engagement with a delivery member shield, a housing, and a delivery member cover having a proximal opening, b) mounting the delivery member cover inside the housing, and c) assembling the cap assembly with the delivery member cover by pushing the remover inside the delivery member cover through the proximal opening of the delivery member cover such that the remover engages with the delivery member cover.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 is an exploded view of an example of a medicament delivery device;
Fig. 2 is a perspective view of components of a cap assembly for the medicament delivery device in Fig. 1;
Fig. 3 shows a longitudinal section of the cap assembly in an assembled state;
Fig. 4 is a close-up view of a proximal portion of the cap assembly shown in Fig. 3;

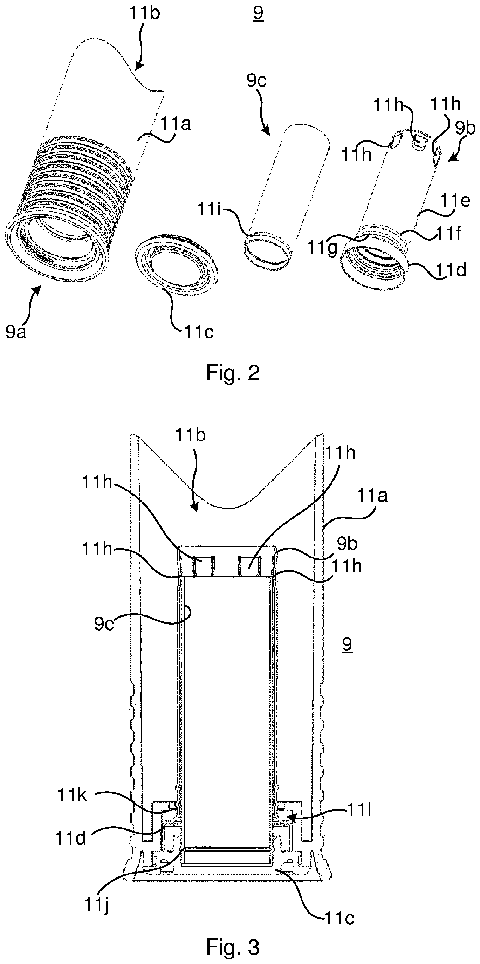

Fig. 3 is a side view of the cap assembly in Fig. 2;

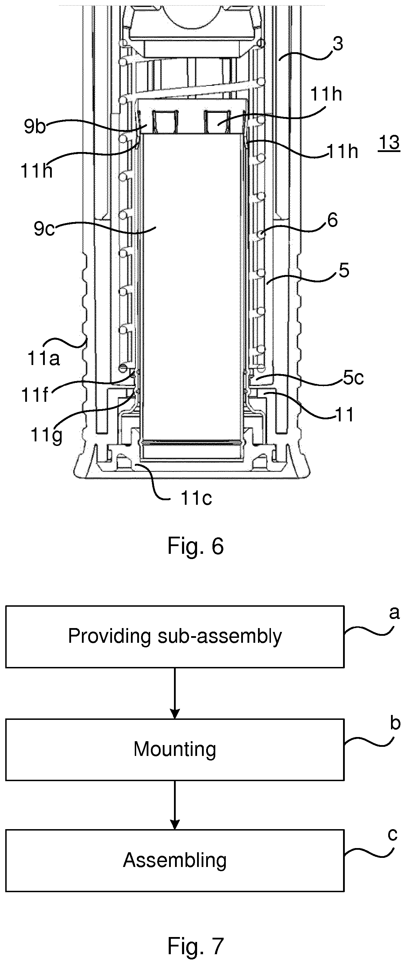

Fig. 6 is a longitudinal section of a sub-assembly including the cap assembly in Fig. 2;
Fig. 7 is a flow chart of a method of assembling a medicament delivery device; and

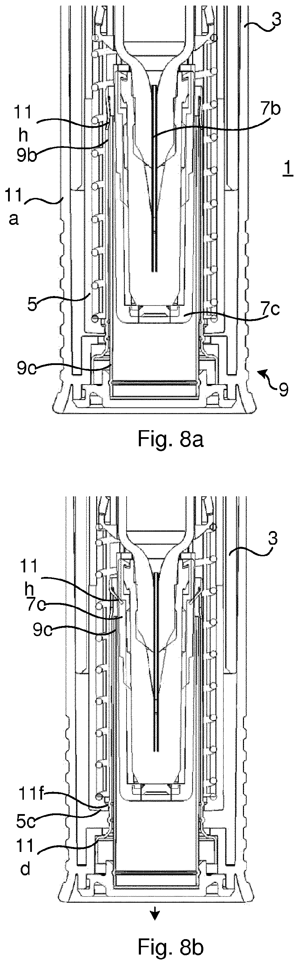

Figs 8a-8c show longitudinal sections of a medicament delivery device and the cap assembly in use, in particular at various stages of removal of the cap assembly from a medicament delivery device.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

The term "proximal end" as used herein, when used in conjunction with a cap assembly, refers to that end of the cap assembly which is farthest from the proximal end of the medicament delivery device, when the cap assembly is properly mounted onto a medicament delivery device. The proximal end of a medicament delivery device is that end which is to be pointed towards the injection site during medicament injection. The same considerations also apply when referring to any component of the cap assembly. The "distal end" is the opposite end relative to the proximal end. With "proximal direction" and, equivalently, "proximally" is meant a direction from the distal end towards the proximal end, along the central axis of the safety mechanism. With "distal direction" or "distally" is meant the opposite direction to "proximal direction". The same definition also applies for the medicament container and any component thereof.

The present disclosure relates to a cap assembly for a medicament delivery device. The cap assembly includes a cap having a tubular body provided with a distal opening, a tubular remover and a tubular remover insert. The cap is configured to receive the remover through the distal opening. The remover is configured to receive the tubular remover insert. The cap, the remover and the remover insert are hence configured to be arranged in a concentric manner. The remover includes a plurality of radially flexible grippers configured to extend radially inwards to engage with a delivery member shield inserted into the remover and remover insert. The tubular remover insert is configured to press the grippers radially outwards when the remover insert is arranged in the remover. In this situation, the grippers will not engage with a delivery member shield received by the remover and the remover insert.

The remover insert is configured to be fixedly attached to the cap. Hereto, the remover insert is prevented from axial displacement relative to the cap.

The remover is configured to be displaceable axially relative to the cap and the remover insert. The remover is in particular displaceable between a first position and a second position relative to the cap and the remover insert. In the first position, the remover insert presses against the grippers, causing the grippers flex radially outwards, as described above. When the remover is displaced from the first position to the second position, the cap and the remover insert are moved axially in the proximal direction relative to the remover, causing the remover insert to move proximally relative to the grippers, proximally beyond the grippers, whereby the grippers are released from being pressed radially outwards by the remover insert. The grippers will thus flex radially inwards, towards their default position. The grippers will thereby be able to engage with a delivery member shield received by the tubular remover.

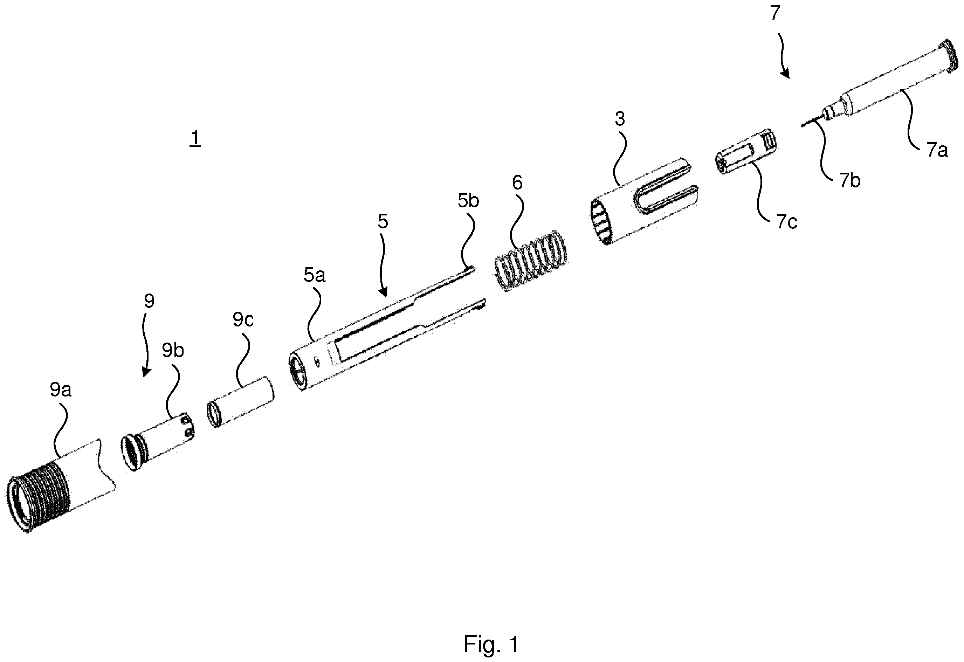

An example of a cap assembly will now be described, initially in the context of a medicament delivery device. Fig. 1 shows an example of a medicament delivery device 1. It is to be noted that some parts of the medicament delivery device 1 depicted in Fig. 1 are not shown, most notably the "power pack", which is an assembly that is to be installed at a distal end of the medicament delivery device, and which contains the drive mechanism for medicament expulsion from a medicament container of the medicament delivery device 1.

Medicament delivery device 1 comprises a housing or body 3, of which for illustrative purposes only a proximal portion is shown, a proximally biased delivery member cover 5 which has a tubular proximal portion 5a and a distal end 5b, and a resilient member 6, for example a spring, configured to bias the delivery member cover 5 proximally. The delivery member cover 5 is configured to be displaced relative to the housing 3, from an extended position relative to the housing 3, in which the delivery member cover 5 extends proximally from the housing 3, to a retracted position, in which the delivery member cover 5 has been displaced in the distal direction and further received by the housing 3.

The previously mentioned "power pack" may for example include a plunger rod, and a moveable sleeve which is configured to engage with and cooperate with the distal end 5b of the delivery member cover 5, such that linear displacement of the delivery member cover 5 in the distal direction, i.e. from the extended position to the retracted position, is translated to rotational motion of the movable sleeve, thereby releasing the plunger rod, enabling it to move in the proximal direction. Medicament expulsion may thus be initiated. The "power pack" will not be described in any further detail herein.

The exemplified medicament delivery device 1 also includes a medicament container assembly 7, which includes a medicament container 7a, a delivery member 7b, according to the present example a needle, and a delivery member shield 7c. The delivery member shield 7c may be a flexible needle shield (FNS) or a rigid needle shield (RNS). Rigid needle shields typically comprise a flexible inner member and a rigid outer member configured to receive the flexible inner member.

The medicament delivery device 1 further comprises a cap assembly 9 including a cap 9a, a tubular remover 9b, and a tubular remover insert 9c.

The cap assembly 9 is configured to be mounted to e.g. the housing 3, to enclose the delivery member cover 5.

Fig. 2 shows the cap assembly 9 in more detail. The cap 9a has a tubular body 11a provided with a distal opening 11b forming a channel extending through the entire tubular body 11a. The cap 9a has a base member 11c configured to be assembled with the tubular body 11a, and defining a proximal end of the cap 9a. The proximal end opening of the cap 9a is hence closable by means of the base member 11c.

The tubular body 11a is configured to receive the remover 9b. The remover 9b is configured to receive the remover insert 9c. The remover insert 9c is configured to receive the delivery member shield 7c. The remover 9b is designed to extend beyond the remover insert 9c in the distal direction, when the remover insert 9c is arranged inside the remover 9b.

The remover 9b has a remover body which has a base that is wider than the remainder of the remover body. The base forms the proximal end portion of the remover 9b and forms a flange 11d.

The exemplified remover 9b has an outer surface 11e provided with a first radial protrusion 11f above the base or flange 11d, i.e. distally relative to the flange 11d. The first radial protrusion 11f extends circumferentially around the remover 9c, along the outer surface 11e thereof. The first radial protrusion 11f may for example be a circumferentially extending rib.

According to one variation, the outer surface 11e of the remover 9b may be provided with a second radial protrusion 11g above the base or flange 11d, i.e. distally relative to the flange 11d. The second radial protrusion 11g extends circumferentially around the remover 9b, along the outer surface 11e thereof. The second radial protrusion 11g may for example be a circumferentially extending rib. The second radial protrusion 11g may be arranged proximally relative to the first radial protrusion 11f. The second radial protrusion 11g is hence arranged axially between the first radial protrusion 11f and the flange 11d.

The remover 9c furthermore comprises a plurality of grippers 11h, for example prongs, arranged along the circumference of the remover 9b. The grippers 11h are radially flexible and extend radially inwards relative to an inner wall of the remover 9b. The grippers 11h are configured to engage with the delivery member shield 7c when the delivery member shield 7c is received by the remover 9b.

The grippers 11h are located distally from the first radial protrusion 11f. According to one variation a distal end portion of the remover 9b may be provided with the grippers 11h. The grippers 11h may have an increasing radially inwards extension in the proximal direction, as shown in Fig. 2. The distal ends of the grippers 11h may hence be attached to the remover body. Alternatively, the grippers may have an increasing radially inwards extension in the distal direction. In this case, the proximal ends of the grippers may be attached to the remover body.

The exemplified remover insert 9c has a third radial protrusion 11i configured to engage with the cap 9a. The third radial protrusion 11i may extend in the circumferential direction along the outer surface of the remover insert 9c. According to present the example, the proximal end portion of the remover insert 9c is provided with the third radial protrusion 11i.

According to the present example, the base member 11c has a distal opening forming a channel configured to receive the proximal end portion of the remover insert 9c. The inner walls of the base member 11c, forming the channel are provided with a circumferential groove 11j configured to receive the third radial protrusion 11i of the remover insert 9c, as shown in Fig. 3. The remover insert 9c is thus configured to engage with the cap 9a. The remover insert 9c is thereby prevented from axial movement relative to the cap 9a.

Fig. 3 shows the cap assembly 9 in an assembled state. The base member 11c has been mounted to the tubular body 11a of the cap 9a. The base member 11c may be attached to the tubular body 11a by means of engagement means such as snap-lock features, by means of glue, screws, rivets, or by means of any other suitable means for fixing two components to each other.

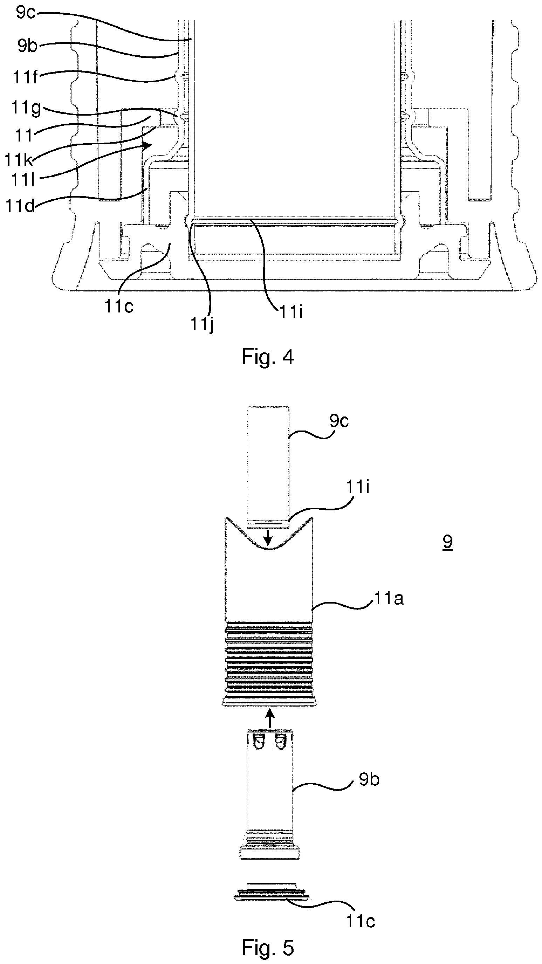

The cap 9a has a flange 11 extending circumferentially inside the tubular body 11a of the cap 9a. The flange has a radial surface 11k. The radial surface 11k and the base member 11c delimit a remover movement space 11l in which the base or flange 11d of the remover 9b is configured to be received, as shown in more detail in Fig. 4. The axial distance from the base member 11c and the radial surface 11k is substantially longer than the axial dimension of the base or flange 11d of the remover. A play is hence formed inside the remover movement space 11l. The flange 11d is thus able to move axially inside the remover movement space 11l. When the flange 11d contacts the base member 11c, the remover 9b is in a first position relative to the cap 9a and the remover insert 9c. When the flange 11d contacts the radial surface 11k, the remover 9b is in a second position relative to the cap 9a and the remover insert 9c.

As can be seen in Fig. 3, in an assembled state, in which the remover 9b is in the first position, the remover insert 9c extends beyond the distal ends of the grippers 11h. The outer diameter of the remover insert 9c essentially corresponds to the inner diameter of the remover 9b. There is hence generally a friction fit between the remover insert 9c and the remover 9b. The outer surface of the remover insert 9c presses the grippers 11h radially outwards from their default radially inwards extending position.

Fig. 5 shows schematically the procedure of assembling the cap assembly 9. Due to the existence of the flange 11d of the remover 9b, the remover 9b is placed into the tubular body 11a of the cap 9a from a bottom end, or proximal end, thereof before the base member 11c is attached to close the proximal end opening of the cap 9a. Next, the remover insert 9c is inserted into the remover 9b and into the tubular body 11a of the cap 9a, from a distal end of the cap 9a, through the distal opening 11b. When inserted into the cap 9a the remover insert 9c is attached to the cap 9a, according to the example by snapping the third radial protrusion 11i into the circumferential groove 11j of the base member 11c.

Fig. 6 shows a sub-assembly 13 of the medicament delivery device 1. The sub-assembly 13 includes the cap assembly 9, the delivery member cover 5 and the housing or body 3.

The delivery member cover 5 is configured to extend into the tubular body 11a of the cap 9a. The delivery member cover 5 is configured to extend coaxially with and radially outside of the remover 9b and the remover insert 9c, but radially inwards of the outer walls of the tubular body 11a.

In the mounted state shown in Fig. 6, the proximal end of the delivery member cover 5 is located just above, i.e. distally from, the flange 11 inside the cap 9a. The first radial protrusion 11f is arranged inside the delivery member cover 5, distally from a proximal flange 5c of the delivery member cover 5. The first radial protrusion 11f will thus engage with the delivery member cover 5 when the cap assembly 9 is moved in the proximal direction, away from the housing 3, initially causing the remover 9b to maintain its axial position in which it engages with the delivery member cover 5, while the remover insert 9c slides in the proximal direction relative to the remover 9b. The function of the cap assembly 9 will be described in more detail with reference to Figs 8a-8c.

The second radial protrusion 11g is located outside of the delivery member cover 5, proximally from the proximal end of the delivery member cover 5.

Fig. 7 shows a method of assembling the medicament delivery device 1, or, alternatively a method of assembling the sub-assembly 13.

In a step a) the sub-assembly 13 comprising the cap assembly 9, the housing 3 and the delivery member cover 5 is provided.

In a step b) the delivery member cover 5 is mounted inside the housing 3.

In a step c) the cap assembly 9 is assembled with the delivery member cover 5 by pushing the remover 9b inside the delivery member cover 5 through a proximal opening of the delivery member cover 5 such that the remover 9b engages with the delivery member cover 5. In particular, the first radial protrusion 11f is moved inside the delivery member cover 5 for engagement with the delivery member cover 5. The remover 9b is placed in the first position relative to the remover insert 9c by ensuring that the flange 11d of the remover 9b contacts the base member 11c.

Subsequently, the medicament container assembly 7 may be placed inside the housing 3, through a distal end of the housing 3. The delivery member 7b, and the delivery member shield 7c enclosing the delivery member 7b will thereby be received inside the remover insert 9c and thus inside the remover 9b. Since the remover 9b is in the first position relative to the remover insert 9c, the grippers 11h are pressed radially outwards by the remover insert 9c. At a later stage, the "power pack" may be mounted from a distal end of the housing 3.

Turning now to Figs 8a-8c, the procedure of removing the cap assembly and the delivery member shield 7c from the medicament delivery device 1 will now be described in more detail.

In Fig. 8a, the cap assembly 9 is fully assembled with the housing 3, and the remover insert 9c presses the grippers 11h radially outwards. The remover 9b is in the first position relative to the remover insert 9c.

In Fig. 8b, the cap assembly 9 removal has commenced and the cap assembly 9 has been moved in the proximal direction relative to the housing 3, as shown by the downwards arrow. The first radial protrusion 11f engages with the proximal flange 5c of the delivery member cover 5, causing the remover 9b to maintain a fixed axial position relative to the delivery member cover 5 while the cap 9a and the remover insert 9c are moved away from the housing 3. This relative movement between the remover 9b and the remover insert 9c is made possible by the play inside the remover movement space ill. By this motion, the remover 9b eventually obtains the second position relative to the remover 9c and the cap 9a. The grippers 11h are thus released in this process, allowing them to flex radially inwards, towards their default position. This causes the grippers 11h to engage with a distal portion of the delivery member shield 7c.

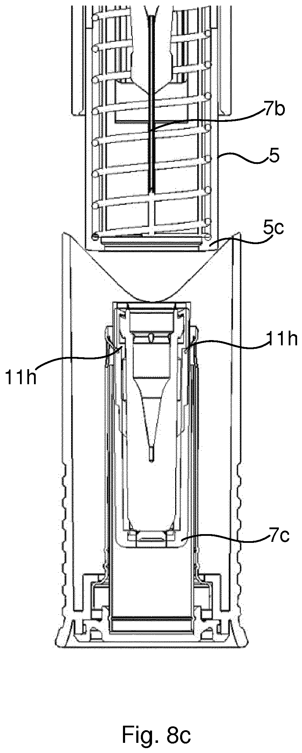

As shown in Fig. 8c, when the cap assembly 9 is pulled further in the proximal direction, with a force greater than what the engagement between the delivery member cover 5 and the first radial protrusion 11f can withstand, the first radial protrusion 11f will eventually move past the proximal flange 5c of the delivery member cover 5 and the cap assembly 9 will be removed from the medicament delivery device 1. Since the grippers 11h engage with the delivery member shield 7c, the delivery member shield 7c is removed concurrently with the cap assembly 9 and is thus removed from the delivery member 7b. The medicament delivery device 1 will as a result be ready for medicament administration.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A sub-assembly (13) for a medicament delivery device (i), which sub-assembly (13) comprises:
a housing (3),
a delivery member cover (5) configured to be received by the housing (3) and configured to move axially relative to the housing (3), and
a cap assembly (9), which cap assembly (9) comprises:
a cap (9a) having a tubular body (11a) provided with a distal opening (11b), the cap (9a) being configured to be mounted to a proximal end of a medicament delivery device (1),
a tubular remover (9b) configured to be received in the distal opening (11b) of the cap (9a), the remover (9b) having flexible radially inwards extending grippers (11h) configured to engage with a delivery member shield (7c), and
a tubular remover insert (9c) configured to be received by the remover (9b) and configured to be fixedly attached to the cap (9a) to prevent axial displacement of the remover insert (9c) relative to the cap (9a),
**characterized in that** the remover (9b) is axially displaceable relative to the cap (9a) and relative to the remover insert (9c), from a first position in which the grippers (11h) are pressed radially outwards by an outer surface of the remover insert (9c), to a second position located distally relative to the first position, in which second position the grippers (11h) are arranged distally beyond the outer surface of the remover insert (9c), enabling the grippers (11h) to flex radially inwards to allow engagement with a delivery member shield (7c), and wherein the cap assembly (9) is configured to be mounted onto a proximal end of the housing (3), and wherein the remover (9b) is configured to be received by the delivery member cover (5).

2. The sub-assembly (13) as claimed in claim 1, wherein the remover (9b) has an outer surface (11e) provided with a first radial protrusion (11f).

3. The sub-assembly (13) as claimed in claim 2, wherein the first radial protrusion (11f) extends in the circumferential direction along the outer surface (11e) of the remover (9b).

4. The sub-assembly (13) as claimed in claim 2 or 3, wherein the outer surface (11e) of the remover is provided with a second radial protrusion (11g).

5. The sub-assembly (13) as claimed in claim 4, wherein the second radial protrusion (11g) is located proximally relative to the first radial protrusion (11f).

6. The sub-assembly (13) as claimed in claim 4 or 5, wherein the second radial protrusion (11g) extends in the circumferential direction along the outer surface (11e) of the remover (9b).

7. The sub-assembly (13) as claimed in any of the preceding claims, wherein the remover (9b) has a proximal end portion provided with a flange (11d).

8. The sub-assembly (13) as claimed in claim 7, wherein the cap (9a) has a base member (11c) defining a proximal end of the cap (9a), and a radial surface (11k) arranged distally from the base member (11c), which base member (11c) and radial surface (11k) delimit a remover movement space (11l), wherein the flange (11d) of the remover (9b) is configured to be arranged in the remover movement space (11l), thereby enabling axial movement of the flange (11d) between the base member (11c) in which the remover (9b) is in the first position and the radial surface (11k) in which the remover (9b) is in the second position.

9. The sub-assembly (13) as claimed in claim 8, wherein the base member (11c) is configured to be attached to the tubular body (11a) of the cap (9a), and wherein the remover insert (9c) is configured to be fixedly attached to the base member (11c).

10. The sub-assembly (13) as claimed in claim 2, wherein the first radial protrusion (11f) is configured to engage with a proximal end portion of the delivery member cover (5) such that proximal movement of the cap (9a) causes distal movement of the remover (9b) relative to the cap (9a), from the first position to the second position thereof.

11. The sub-assembly (13) as claimed in claim 4, , wherein the second radial protrusion (11g) is configured to be arranged proximally from a proximal end of the delivery member cover (5) when the cap assembly (9) is mounted to the delivery member cover (5), to restrict distal movement of the remover (9b) relative to the delivery member cover (5).

12. A medicament delivery device (1) comprising a sub-assembly (13) as claimed in any of claims 1-11.

13. The medicament delivery device (1) as claimed in claim 12, wherein the medicament delivery device (1) further comprising a medicament container assembly (7) inside the housing (3) and a power pack.

14. A method of assembling a medicament delivery device (1), comprising:
a) providing a sub-assembly (13) comprising:
a cap (9a) having a tubular body (11a) provided with a distal opening (11b),
a tubular remover (9b) received in the distal opening (11b) of the cap (9a), the remover (9b) having flexible radially inwards extending grippers (11h) configured to engage with a delivery member shield (7c), and
a tubular remover insert (9c) received by the remover (9b) and fixedly attached to the cap (9a) to prevent axial displacement of the remover insert (9c) relative to the cap (9a),
wherein the remover (9b) is axially displaceable relative to the cap (9a) and relative to the remover insert (9c), from a first position in which the grippers (11h) are pressed radially outwards by an outer surface of the remover insert (9c), to a second position located distally relative to the first position, in which second position the grippers (11h) are arranged distally beyond the outer surface of the remover insert (9c), enabling the grippers (11h) to flex radially inwards to allow engagement with a delivery member shield (7c),
a housing (3),
and a delivery member cover (5) having a proximal opening, **characterized by**
b) mounting the delivery member cover (5) inside and axially moveable to the housing (3), and
c) assembling the cap assembly (9) with the delivery member cover (5) by pushing the remover inside (9b) the delivery member cover (5) through the proximal opening of the delivery member cover (5) such that the remover (9b) engages with the delivery member cover (5).

15. The method of assembling a medicament delivery device (1), as claimed in claim 14, **characterized by:**
inserting a medicament container assembly (7) inside the housing (3) through a distal end of the housing (3),
mounting a power pack from a distal end of the housing (3).

## Patentansprüche

1. Unteranordnung (13) für eine Medikamentenabgabevorrichtung (1), wobei die Unteranordnung (13) umfasst:
ein Gehäuse (3),
eine Abgabeelementabdeckung (5), konfiguriert, um von dem Gehäuse (3) aufgenommen zu werden, und konfiguriert, sich relativ zu dem Gehäuse (3) axial zu bewegen, und
eine Kappenanordnung (9), wobei die Kappenanordnung (9) umfasst:
eine Kappe (9a) mit einem rohrförmigen Körper (11 a), der mit einer distalen Öffnung (11b) versehen ist, wobei die Kappe (9a) konfiguriert ist, um an einem proximalen Ende einer Medikamentenabgabevorrichtung (1) befestigt zu werden,
einen rohrförmigen Entferner (9b), konfiguriert, um in der distalen Öffnung (11b) der Kappe (9a) aufgenommen zu werden, wobei der Entferner (9b) flexible, sich radial nach innen erstreckende Greifer (11h) aufweist, konfiguriert, um mit einem Abgabeelementschild (7c) in Eingriff zu kommen, und
einen rohrförmigen Entfernereinsatz (9c), konfiguriert, um von dem Entferner (9b) aufgenommen zu werden, und konfiguriert, um fest an der Kappe (9a) angebracht zu werden, um axiale Verschiebung des Entfernereinsatzes (9c) relativ zu der Kappe (9a) zu verhindern, **dadurch gekennzeichnet, dass** der Entferner (9b) relativ zu der Kappe (9a) und relativ zu dem Entfernereinsatz (9c) von einer ersten Position, in der die Greifer (11h) durch eine Außenoberfläche des Entfernereinsatzes (9c) radial nach außen gedrückt werden, in eine zweite Position axial verschiebbar ist, die sich relativ zu der ersten Position distal befindet, wobei die Greifer (11h) in der zweiten Position distal über die Außenoberfläche des Entfernereinsatzes (9c) hinaus angeordnet sind, was den Greifern (11h) ermöglicht, sich radial nach innen zu biegen, um Eingriff mit einem Abgabeelementschild (7c) zu erlauben, und wobei die Kappenanordnung (9) konfiguriert ist, um an einem proximalen Ende des Gehäuses (3) befestigt zu werden, und wobei der Entferner (9b) konfiguriert ist, um von der Abgabeelementabdeckung (5) aufgenommen zu werden.

2. Unteranordnung (13) nach Anspruch 1, wobei der Entferner (9b) eine Außenoberfläche (11e) aufweist, die mit einem ersten radialen Vorsprung (11f) versehen ist.

3. Unteranordnung (13) nach Anspruch 2, wobei sich der erste radiale Vorsprung (11f) in der Umfangsrichtung entlang der Außenoberfläche (11e) des Entferners (9b) erstreckt.

4. Unteranordnung (13) nach Anspruch 2 oder 3, wobei die Außenoberfläche (11e) des Entferners mit einem zweiten radialen Vorsprung (11g) versehen ist.

5. Unteranordnung (13) nach Anspruch 4, wobei sich der zweite radiale Vorsprung (11g) relativ zu dem ersten radialen Vorsprung (11f) proximal befindet.

6. Unteranordnung (13) nach Anspruch 4 oder 5, wobei sich der zweite radiale Vorsprung (11g) in der Umfangsrichtung entlang der Außenoberfläche (11e) des Entferners (9b) erstreckt.

7. Unteranordnung (13) nach einem der vorstehenden Ansprüche, wobei der Entferner (9b) einen mit einem Flansch (11d) versehenen proximalen Endabschnitt aufweist.

8. Unteranordnung (13) nach Anspruch 7, wobei die Kappe (9a) ein Basiselement (11c), das ein proximales Ende der Kappe (9a) definiert, und eine radiale Oberfläche (11k) aufweist, die von dem Basiselements (11c) distal angeordnet ist, wobei das Basiselement (11c) und die radiale Oberfläche (11k) einen Entfernerbewegungsraum (11l) begrenzen, wobei der Flansch (11d) des Entferners (9b) konfiguriert ist, um in dem Entfernerbewegungsraum (11l) angeordnet zu werden, wodurch axiale Bewegung des Flansches (11d) zwischen dem Basiselement (11c), in dem der Entferner (9b) in der ersten Position vorliegt, und der radialen Oberfläche (11k), in der der Entferner (9b) in der zweiten Position vorliegt, ermöglicht wird.

9. Unteranordnung (13) nach Anspruch 8, wobei das Basiselement (11c) konfiguriert ist, um an dem rohrförmigen Körper (11a) der Kappe (9a) angebracht zu werden, und wobei der Entfernereinsatz (9c) konfiguriert ist, um fest an dem Basiselement (11c) angebracht zu werden.

10. Unteranordnung (13) nach Anspruch 2, wobei der erste radiale Vorsprung (11f) konfiguriert ist, um mit einem proximalen Endabschnitt der Abgabeelementabdeckung (5) in Eingriff zu kommen, sodass proximale Bewegung der Kappe (9a) distale Bewegung des Entferners (9b) relativ zu der Kappe (9a) von der ersten Position in die zweite Position davon bewirkt.

11. Unteranordnung (13) nach Anspruch 4, wobei der zweite radiale Vorsprung (11g) konfiguriert ist, um von einem proximalen Ende der Abgabeelementabdeckung (5) proximal angeordnet zu werden, wenn die Kappenanordnung (9) an der Abgabeelementabdeckung (5) befestigt ist, um distale Bewegung des Entferners (9b) relativ zu der Abgabeelementabdeckung (5) einzuschränken.

12. Medikamentenabgabevorrichtung (1), umfassend eine Unteranordnung (13) nach einem der Ansprüche 1-11.

13. Medikamentenabgabevorrichtung (1) nach Anspruch 12, wobei die Medikamentenabgabevorrichtung (1), ferner umfassend eine Medikamentenbehälteranordnung (7) im Inneren des Gehäuses (3) und ein Power Pack.

14. Verfahren zur Montage einer Medikamentenabgabevorrichtung (1), das Folgendes umfasst:
a) Bereitstellen einer Unteranordnung (13), umfassend:
eine Kappe (9a) mit einem rohrförmigen Körper (11a), der mit einer distalen Öffnung (11b) versehen ist,
einen rohrförmigen Entferner (9b), der in der distalen Öffnung (11b) der Kappe (9a) aufgenommen ist, wobei der Entferner (9b) flexible, sich radial nach innen erstreckende Greifer (11h) aufweist, konfiguriert, um mit einem Abgabeelementschild (7c) in Eingriff zu kommen, und
einen rohrförmigen Entfernereinsatz (9c), der von dem Entferner (9b) aufgenommen und fest an der Kappe (9a) angebracht wird, um axiale Verschiebung des Entfernereinsatzes (9c) relativ zu der Kappe (9a) zu verhindern,
wobei der Entferner (9b) relativ zu der Kappe (9a) und relativ zu dem Entfernereinsatz (9c) aus einer ersten Position, in der die Greifer (11h) durch eine Außenoberfläche des Entfernereinsatzes (9c) radial nach außen gedrückt werden, axial in eine zweite Position verschiebbar ist, die sich relativ zu der ersten Position distal befindet, wobei die Greifer (11h) in der zweiten Position distal über die Außenoberfläche des Entfernereinsatzes (9c) hinaus angeordnet sind, was den Greifern (11h) ermöglicht, sich radial nach innen zu biegen, um Eingriff mit einem Abgabeelementschild (7c) zu erlauben,
ein Gehäuse (3),
und eine Abgabeelementabdeckung (5) mit einer proximalen Öffnung,
**gekennzeichnet durch**
b) Befestigen der Abgabeelementabdeckung (5) im Inneren und axial bewegbar zu dem Gehäuse (3) und
c) Montieren der Kappenanordnung (9) mit der Abgabeelementabdeckung (5) durch Schieben des Entferners ins Innere (9b) der Abgabeelementabdeckung (5) durch die proximale Öffnung der Abgabeelementabdeckung (5), sodass der Entferner (9b) mit der Abgabeelementabdeckung (5) in Eingriff kommt.

15. Verfahren zur Montage einer Medikamentenabgabevorrichtung (1) nach Anspruch 14, **gekennzeichnet durch:**
Einsetzen einer Medikamentenbehälteranordnung (7) ins Innere des Gehäuses (3) durch ein distales Ende des Gehäuses (3),
Befestigen eines Power Packs von einem distalen Ende des Gehäuses (3).

## Revendications

1. Sous-ensemble (13) pour un dispositif d'administration de médicament (1), lequel sous-ensemble (13) comprend :
un logement (3),
un couvercle d'élément d'administration (5) configuré pour être reçu par le logement (3) et configuré pour se déplacer axialement par rapport au logement (3), et
un ensemble capuchon (9), lequel ensemble capuchon (9) comprend :
un capuchon (9a) ayant un corps tubulaire (11a) pourvu d'une ouverture distale (11b), le capuchon (9a) étant configuré pour être monté à une extrémité proximale d'un dispositif d'administration de médicament (1),
un extracteur tubulaire (9b) configuré pour être reçu dans l'ouverture distale (11b) du capuchon (9a), l'extracteur (9b) ayant des organes de préhension (11h) flexibles s'étendant radialement vers l'intérieur configurés pour venir en prise avec une protection d'élément d'administration (7c), et
un insert d'extracteur tubulaire (9c) configuré pour être reçu par l'extracteur (9b) et configuré pour être attaché fixement au capuchon (9a) pour empêcher un déplacement axial de l'insert d'extracteur (9c) par rapport au capuchon (9a), **caractérisé en ce que** l'extracteur (9b) est axialement déplaçable par rapport au capuchon (9a) et par rapport à l'insert d'extracteur (9c), depuis une première position dans laquelle les organes de préhension (11h) sont pressés radialement vers l'extérieur par une surface externe de l'insert d'extracteur (9c), jusqu'à une deuxième position située de façon distale par rapport à la première position, dans laquelle deuxième position les organes de préhension (11h) sont agencés de façon distale au-delà de la surface externe de l'insert d'extracteur (9c), permettant aux organes de préhension (11h) de fléchir radialement vers l'intérieur pour permettre une mise en prise avec une protection d'élément d'administration (7c), et dans lequel l'ensemble capuchon (9) est configuré pour être monté sur une extrémité proximale du logement (3), et dans lequel l'extracteur (9b) est configuré pour être reçu par le couvercle d'élément d'administration (5).

2. Sous-ensemble (13) tel que revendiqué dans la revendication 1, dans lequel l'extracteur (9b) a une surface externe (11e) pourvue d'une première partie saillante radiale (11f).

3. Sous-ensemble (13) tel que revendiqué dans la revendication 2, dans lequel la première partie saillante radiale (11f) s'étend dans la direction circonférentielle le long de la surface externe (11e) de l'extracteur (9b).

4. Sous-ensemble (13) tel que revendiqué dans la revendication 2 ou 3, dans lequel la surface externe (11e) de l'extracteur est pourvue d'une deuxième partie saillante radiale (11g).

5. Sous-ensemble (13) tel que revendiqué dans la revendication 4, dans lequel la deuxième partie saillante radiale (11g) est située de façon proximale par rapport à la première partie saillante radiale (11f).

6. Sous-ensemble (13) tel que revendiqué dans la revendication 4 ou 5, dans lequel la deuxième partie saillante radiale (11g) s'étend dans la direction circonférentielle le long de la surface externe (11e) de l'extracteur (9b).

7. Sous-ensemble (13) tel que revendiqué dans l'une quelconque des revendications précédentes, dans lequel l'extracteur (9b) a une partie d'extrémité proximale pourvue d'un rebord (11d).

8. Sous-ensemble (13) tel que revendiqué dans la revendication 7, dans lequel le capuchon (9a) a un élément de base (11c) définissant une extrémité proximale du capuchon (9a), et une surface radiale (11k) agencée de façon distale par rapport à l'élément de base (11c), lequel élément de base (11c) et surface radiale (11k) délimitent un espace de mouvement d'extracteur (11l), dans lequel le rebord (11d) de l'extracteur (9b) est configuré pour être agencé dans l'espace de mouvement d'extracteur (11l), ce qui permet un mouvement axial du rebord (11d) entre l'élément de base (11c) dans lequel l'extracteur (9b) est dans la première position et la surface radiale (11k) dans lequel l'extracteur (9b) est dans la deuxième position.

9. Sous-ensemble (13) tel que revendiqué dans la revendication 8, dans lequel l'élément de base (11c) est configuré pour être attaché au corps tubulaire (11a) du capuchon (9a), et dans lequel l'insert d'extracteur (9c) est configuré pour être attaché fixement à l'élément de base (11c).

10. Sous-ensemble (13) tel que revendiqué dans la revendication 2, dans lequel la première partie saillante radiale (11f) est configurée pour venir en prise avec une partie d'extrémité proximale du couvercle d'élément d'administration (5) de telle sorte qu'un mouvement proximal du capuchon (9a) amène un mouvement distal de l'extracteur (9b) par rapport au capuchon (9a), depuis la première position jusqu'à la deuxième position de celui-ci.

11. Sous-ensemble (13) tel que revendiqué dans la revendication 4, , dans lequel la deuxième partie saillante radiale (11g) est configurée pour être agencée de façon proximale à partir d'une extrémité proximale du couvercle d'élément d'administration (5) lorsque l'ensemble capuchon (9) est monté au couvercle d'élément d'administration (5), pour limiter un mouvement distal de l'extracteur (9b) par rapport au couvercle d'élément d'administration (5).

12. Dispositif d'administration de médicament (1) comprenant un sous-ensemble (13) tel que revendiqué dans l'une quelconque des revendications 1 à 11.

13. Dispositif d'administration de médicament (1) tel que revendiqué dans la revendication 12, dans lequel le dispositif d'administration de médicament (1) comprenant en outre un ensemble récipient de médicament (7) à l'intérieur du logement (3) et un bloc d'alimentation.

14. Procédé d'assemblage d'un dispositif d'administration de médicament (1), comprenant :
a) la fourniture d'un sous-ensemble (13) comprenant :
un capuchon (9a) ayant un corps tubulaire (11a) pourvu d'une ouverture distale (11 b),
un extracteur tubulaire (9b) reçu dans l'ouverture distale (11b) du capuchon (9a), l'extracteur (9b) ayant des organes de préhension (11h) flexibles s'étendant radialement vers l'intérieur configurés pour venir en prise avec une protection d'élément d'administration (7c), et
un insert d'extracteur tubulaire (9c) reçu par l'extracteur (9b) et attaché fixement au capuchon (9a) pour empêcher un déplacement axial de l'insert d'extracteur (9c) par rapport au capuchon (9a),
dans lequel l'extracteur (9b) est axialement déplaçable par rapport au capuchon (9a) et par rapport à l'insert d'extracteur (9c), depuis une première position dans laquelle les organes de préhension (11h) sont pressés radialement vers l'extérieur par une surface externe de l'insert d'extracteur (9c), jusqu'à une deuxième position située de façon distale par rapport à la première position, dans laquelle deuxième position les organes de préhension (11h) sont agencés de façon distale au-delà de la surface externe de l'insert d'extracteur (9c), permettant aux organes de préhension (11h) de fléchir radialement vers l'intérieur pour permettre une mise en prise avec une protection d'élément d'administration (7c),
un logement (3),
et un couvercle d'élément d'administration (5) ayant une ouverture proximale, **caractérisé par**
b) le montage du couvercle d'élément d'administration (5) à l'intérieur du et mobile axialement par rapport au logement (3), et
c) l'assemblage de l'ensemble capuchon (9) avec le couvercle d'élément d'administration (5) en poussant l'extracteur à l'intérieur (9b) du couvercle d'élément d'administration (5) à travers l'ouverture proximale du couvercle d'élément d'administration (5) de telle sorte que l'extracteur (9b) vient en prise avec le couvercle d'élément d'administration (5).

15. Procédé d'assemblage d'un dispositif d'administration de médicament (1), tel que revendiqué dans la revendication 14, **caractérisé par** :
l'insertion d'un ensemble récipient de médicament (7) à l'intérieur du logement (3) à travers une extrémité distale du logement (3),
le montage d'un bloc d'alimentation à partir d'une extrémité distale du logement (3).
